(19) Europäisches Patentamt
European Patent Office
Office européen des brevets

(11) EP 0 529 264 B1

(12) EUROPEAN PATENT SPECIFICATION

(45) Date of publication and mention of the grant of the patent: **10.06.1998 Bulletin 1998/24**

(51) Int. Cl.$^6$: **A61K 6/083**

(21) Application number: **92111787.5**

(22) Date of filing: **10.07.1992**

# (54) Self-lubricating abrasion resistant material and products for use in dentistry

Selbstschmierendes abriebfestes Material und Gegenstände zum Gebrauch in der Zahnheilkunde

Matériau et produits autolubrifiants résistants à l'abrasion pour utilisation dans l'art dentaire

(84) Designated Contracting States:
**AT BE CH DE FR GB IT LI NL**

(30) Priority: **12.07.1991 US 729018**

(43) Date of publication of application:
**03.03.1993 Bulletin 1993/09**

(60) Divisional application:
**97120214.8 / 0 829 252**

(73) Proprietor:
**DENTSPLY INTERNATIONAL, INC.**
**York, Pennsylvania 17405 (US)**

(72) Inventor: **Liu, Andrew T. C.**
**York, PA 17404 (US)**

(74) Representative:
**Wächtershäuser, Günter, Prof. Dr.**
**Patentanwalt,**
**Tal 29**
**80331 München (DE)**

(56) References cited:

| | |
|---|---|
| **EP-A- 0 358 195** | **WO-A-82/02556** |
| **DD-A- 258 641** | **DE-A- 3 038 153** |

- **DATABASE WPIL Week 8334, Derwent Publications Ltd., London, GB; AN 83-743786 & JP-A-58 118 749 (DAINIPPON PRINTING KK)**

EP 0 529 264 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The invention relates to an artificial tooth dental prosthesis

Artificial teeth should exhibit certain physical and physiochemical characteristics to be suitable for use. They should be hard and resistant to chipping, durable, and stable to solvents, water, and heat. In addition, they should be of an aesthetically acceptable color, i.e., close to that of natural teeth, or be amenable to artificial coloration. The teeth should not cause excessive wear to opposing natural or artificial teeth, should not wear out of occlusion, and should be capable of being bonded firmly to supportive structures. They should also be amenable to ordinary means of physical shaping, grinding, and polishing, so as to minimize production costs.

Various metals and ceramics as used in the formation of prior art artificial teeth and other dental appliances possess certain inherent deficiencies which lessen their desirability in dental applications. Thus, the metallic color of gold, amalgam, and other metallic species serves as an aesthetic detraction to the wearer of appliances made therefrom. In addition, the high cost of most noble metals from which many such appliances are commonly made leads to a cost consideration whenever their use is contemplated. Ceramic materials, another common alternative, are often difficult to form into acceptable shapes, and may tend to be abrasive and aesthetically unpleasant subsurfaces upon the physical wearing of surface layers. Such materials are also difficult to polish satisfactorily. These reasons together with factors related to cost, to consumer preference, to the technical skills of dental practitioners, and to convenience have motivated a search for alternative compositions suitable for the construction of dental appliances, inlays, onlays, crown and bridge material, artificial teeth and the like.

Of the presently available organic compositions used for the construction of artificial teeth, most are composed of acrylics, often crosslinked by polyfunctional moieties.

DESCRIPTION OF THE PRIOR ART

As used herein "self-lubricating material" means a material which is adapted to increase the lubricity of a product surface. Preferably self-lubricating material provides the product surface with a reduced kinetic coefficient of friction.

As used heerein ASTM D 1894-78 is carried out using water and the same materials.

It is to be understood that the term "bisphenol-A" is commonly utilized in the art to indicate the chemical compound 2,2-bis(4-hydroxyphenyl)propane. It is also to be understood that the term "bis-GMA" is commonly used to indicate the chemical compound 2,2-bis(4-(2-hydroxy-3-methacryloxypropoxy)phenyl)propane, otherwise referred to as "digycidyl methacrylate of bisphenol-A."

Dentsply U.S. Patents 4,396,476, 4,396,377 and 4,698,373 (the disclosures of which are incorporated herein by reference) disclose interpenetrating network teeth but do not disclose self-lubricating abrasion resistant hardenable compositions as required by applicants' invention.

Thornton U. S. Patent 2,345,305 discloses making artificial teeth comprised of different plastic materials for the face ("enamel") and body portions. Note Figure 17, and page 4, column 2, lines 21-24. Another composite plastic tooth structure is disclosed by Cornell U. S. Patent 3,488,846.

The Rosenkranz et al. U. S. Patent 3,928,299 discloses an organic homopolymer or random copolymer containing urethane groups.

Michl et al. in U. S. patents 4,267,097 and 4,281,991 (the disclosures of which are incorporated herein by reference) disclose artificial teeth prepared from (a) particle/bead PMMA, (b) a liquid monomer such as the adduct of hydroxy-methacrylates and diisocyanates or difunctional esters of (meth)acrylic acids or mixtures thereof, and (c) micro-fine inorganic fillers. Michl et al do not disclose self-lubricating abrasion resistant hardenable compositions as requred by Applicants' invention.

Walkowiak et al. in U. S. Patents 4,308,190 and 4,369,262 disclose dental paste materials of a polymerizable acrylic ester, a crosslinked bead polymer, and a particulate inorganic filler and do not disclose self-lubricating abrasion resistant hardenable compositions, or interpenetrating network compositions for making artificial teeth as required by Applicants' invention.

Simpson in U.S. Patent 4,361,676 discloses a sag-resistant, pumpable composition comprising a liquid material dispersed throughout a synthetic, continuous cross-linked polymer matrix.

Wright et al. in U.S. Patent 4,598,111 disclose the use of various divinyl compounds, including divinyl dimethyl silane (column 6, line 35) as a crosslinking agent for (meth)-acrylate monomer systems. Other patents of this general type include, for example, Kohno et al. U. S. Patent 4,761,436; dimethyldivinylsilane as a co-monomer; column 3, line 29); Feinberg et al. U. S. Patent 4,894,315; column 3, lines 37-38); Fryd et al. U. S. Patent 4,956,252; column 5, lines 43-44); and Kafka et al. U. S. Patent 4,970,037; column 9, lines 16-17).

Yamazaki et al. in U. S. Patent 4,826,893 disclose a dental composition comprising (a) a siloxane polymer, (b) a monomer copolymerizable with the siloxane polymer, (c) a polymerization catalyst, e.g. benzoyl peroxide, and optionally, (d) a filler.

Laundry in U.S. Patent No. 3,084,436 discloses soft dental materials manufactured from mixtures of methacrylate monomers. Monofunctional esters together with vinyl-acetate or vinyl stearate are crosslinked with polyfunctional esters of acrylic or methacrylic acid. The resulting product is disclosed as being three dimensionally crosslinked.

Graham et al. in U.S. Patent No. 3,087,875 disclose preparation of graft copolymers. Alkyl methacrylate and analogous polymers are dissolved in monomers such as alkyl acrylates, alkyl thioacrylates, and N-vinyl lactams. The monomers are subsequently grafted to the preformed polymers via photochemical initiation.

Cornell in U.S. Patent No. 3,427,274 discloses hard-enable materials formed from a mixture of methyl methacrylate homopolymer and styrene-butadiene copolymer latex coated with methyl methacrylate polymer which may be incorporated in a methacrylate-crosslinking agent composition to form hardenable compositions.

Chang in U.S. Patent No. 3,452,437 discloses a dental restorative material formed from the "diglycidyl methacrylate of bisphenol-A" (bis-GMA) to which a quantity of methyl methacrylate may be added.

Bruckmann et al. in U.S. Patent No. 3,468,977 disclose the formulation of dental compositions from a mixture of a polymer and a monomer. The preformed uncrosslinked polymer beads are allowed to swell with monomer which may contain a crosslinking agent. Acrylic materials may be used for both the monomer and the polymer.

Petner in U.S. patent No. 3,470,615, teaches the formulation of a material suitable for use in the construction of dental appliances. A mixture of an uncrosslinked homopolymer and crosslinked copolymer is dissolved in a liquid polyglycol dimethacrylate to form a suspension which may be brushed on a substratum and subsequently hardened by heat to build up layers of polymeric material. A similar teaching may be found in U.S. Patent No. 3,471,596, also to Petner et al. A thick liquid is provided which is useful in the building up of dental crowns and the like. The difunctional monomer may contain various thickening agents including poly(methyl methacrylate). In some embodiments, the poly(methyl methacrylate) may be supplemented with additional polymer which may be partially crosslinked with allyl methacrylate.

Lee in U.S. Patent No. 3,539,533 discloses a filling material including a monomer solution filled with inorganic particulate filler. The monomer solution may be a mixture of methacrylate monomers containing bisphenol-A dimethacrylate.

Talyor in U.S. Patent No. 3,597,389 discloses polyfunctional methacrylate monomers, including "bis-phenol-A glycidyl dimethacrylate"(bis-GMA), polymerized with an inorganic filler to yield dental compositions.

Waller in U.S. Patent No. 3,629,187 discloses the use of the isocyanate or diisocyanate adducts of bisphenol-A type compounds. These adducts are employed together with various inorganic fillers and liquid monomers to form liquid or paste compositions which are polymerizable either thermally or photochemically.

Dougherty in U.S. patent No. 3,647,498 discloses dental compositions which are composed of liquid-solid mixtures. The solid phase is an acrylate or methacrylate polymer in bead form.

Logemann in U.S. Patent 3,649,608 discloses dental compositions which comprise solid bead polymers or copolymers of methacrylate type materials.

Lee in U.S. Patent No. 3,751,399 discloses compositions for dental use comprising aromatic and alicyclic polyacrylates which are mixed together with other polyacrylate compounds especially those containing bisphenol-A structures.

Sperling in U.S. Patent No. 3,833,404 discloses elastomers, especially acrylates, urethanes, butadienes, natural rubbers, and polyvinyl alcohol, are formulated which possess interpenetrating polymeric network type structures. These materials are disclosed as being "hard", but are used as vibration and sound damping insulators.

Highgate in U.S. Patent No. 3,961,379 discloses an article manufactured from a crosslinked polymer which is swollen with a monomer containing a crosslinking agent.

None of the foregoing patents discloses or suggests the novel hardenable self-lubricating abrasion resistant compositions of the present invention.

## OBJECTS OF THE INVENTION

It is an object of the invention to provide an artificial tooth dental prosthesis which is wear resistant and self-lubricating across its entire cross-section.

This object is achieved by the artificial tooth according to Claim 1.

Preferably, the artificial tooth dental prosthesis comprises a self-lubricating abrasion resistant material having an outer surface having a reduced kinetic coefficient of friction.

Preferably, the self-lubricating abrasion resistant monomeric material is adapted to crosslink the matrix material.

Preferably, the artificial tooth comprises divinyldimethyl silane or divinyldimethyl siloxane.

Preferably, the artificial tooth is made from polyethylene particles having a molecular weight of at least 1,000,000, and a particle size less than 80 microns whereby the particles are entangled in an interpenetrating network.

Preferably, the artificial tooth has an enamel coating which includes silicon containing material which is polymerizable within a matrix forming polymer.

## BRIEF DESCRIPTION OF THE DRAWINGS

FIGURE 1 is a cross-sectional side view of an anterior interpenetrating network tooth in accordance with the invention.

FIGURE 2 is a front view of an anterior interpenetrating network tooth in accordance with the invention.

FIGURE 3 is a cross-sectional side view of an posterior interpenetrating network tooth in accordance with the invention.

FIGURE 4 is a front view of an posterior interpenetrating network tooth in accordance with the invention.

## DETAILED DESCRIPTION OF THE INVENTION

The invention is now discussed with more particular reference to FIGURES 1-4 in which like numerals refer to the same component. FIGURES 1 and 2 show an artificial anterior tooth 10 having a tooth body 12 and an enamel coating 14. Tooth 10 includes ridge lap 16, lingual face 18 and buckle face 20. Optionally tooth 10 includes intermediate layer 22. Tooth body 12 is substantially opaque and includes high molecular weight polyethylene particles in an interpenetrating network. Enamel 14 is translucent and includes a silicon containing monomer or oligomer in an interpenetrating network. Intermediate layer 22 includes pigment and high molecular weight polyethylene particles in an interpenetrating network.

FIGURES 3 and 4 show an artificial posterior tooth 30 having a tooth body 32 and an enamel coating 34. Tooth 30 includes ridge lap 36, buckle face 38, occlusal face 40 and lingual face 42. Tooth body 32 includes high molecular weight polyethylene particles in an interpenetrating network. Enamel 34 includes high molecular weight polyethylene particles in an interpenetrating network.

Teeth made in accordance with the invention are made entirely or in part from self-lubricating abrasion resistant material added in the form of particles, fibers and/or monomer adapted to interpolymerize with matrix material, monomers and oligomers. Generally, the smaller the particles of high molecular weight polyethylene the better. However, very small particle sizes are expensive. Most preferably, the polyethylene particles are micronsized particles of ultra-high molecular weight polyethylene that have been functionalized by oxidation in the presence of a fluorine gas.

## SELF-LUBRICATING MATERIALS

Preferably the self-lubricating abrasion resistant material includes a polymer of the general formula:

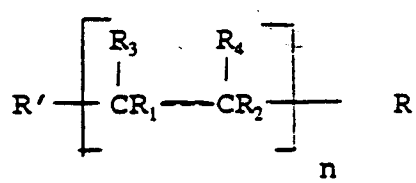

wherein n is an integer from 100 to 1,000,000,

--- is a single or a double bond,

when --- is a double bond R, R', $R_1$ and $R_2$ independently are hydrogen, fluorine or a lower alkyl of from 1 to 6 carbons, and $R_3$ and $R_4$ are not present,

when --- is a single bond R, R', $R_1$, $R_2$, $R_3$ and $R_4$ independently are hydrogen, fluorine or a lower alkyl of from 1 to 6 carbons.

## SILICON CONTAINING SELF-LUBRICATING COMPOUNDS

In a preferred embodiment of the invention a tooth has an enamel coating which includes a silicon containing self-lubricating compound. Silicon containing self-lubricating compounds, useful as self-lubricating abrasion resistant monomeric material include a compound of the general formula:

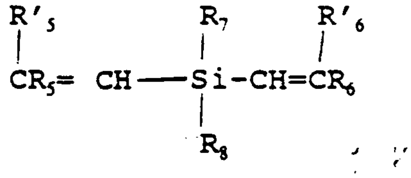

or

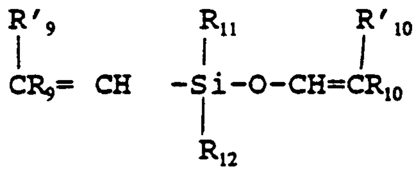

wherein $R_5$, $R'_5$, $R_6$, $R'_6$, $R_7$, $R_8$, $R_9$, $R'_9$, $R_{10}$, $R'_{10}$', $R_{11}$, and $R_{12}$ independently are hydrogen or a lower alkyl of from 1 to 6 carbons. Most preferably the abrasion resistant monomeric material is divinyldimethyl silane or divinyldimethyl siloxane.

DENTAL USES

Teeth and other dental prostheses which are prepared from hardenable dental compositions in accordance with a preferred embodiment of the invention include polymerizable self-lubricating on the outer faces and preferably incisal edge portions thereof, i.e. the "enamel", and include solid self-lubricating material in the body portions. However, teeth in accordance with the invention may be made with solid and/or polymerizable self-lubricating throughout.

In general, the novel compositions of this invention are useful for the formation, construction and repair of dental appliances, artificial teeth, oral prosthetics, and similar articles. In addition, these compositions may be utilized for the filling of teeth, and for the surface coating thereof either to effect adhesion with oral prostheses, or to protect natural teeth from erosion, damage or decay.

The hardenable dental compositions of a preferred interpenetrating network embodiment of the invention includes a blend of powder or liquid components which, when combined in certain proportions and permitted to age or mature to produce a precursor blend that is moldable into prosthetic teeth and other dental devices. The precursor blend is formed by combining polymer and monomer and polymerizing. In another preferred embodiment of the invention precursor blends formed by combining a crosslinked polymer with a crosslinking monomer and/or oligomer, a monomer, and an optional uncrosslinked polymer and/or an initiator and by allowing the combination to age or mature. The crosslinked polymer is in the form of discrete particles having average diameters ranging form about 0.001 micron to about 500 microns. Preferably, at least 50% by weight of said particles have diameters less than about 150 microns, and more preferably, less than 100 microns. A mixture of two or more different crosslinked polymers may be used. A characteristic of the crosslinked polymer is that it will be insoluble in, but will be swollen by the liquid components used in the preparation of the precursor blend. Uncrosslinked polymer may be characterized as being capable of dissolving in or being dispersed by the liquid components of the blend. The liquid polymerizable monomer component of the compositions of the invention is a monomer having the capacity to dissolve or disperse such uncrosslinked polymer, dissolve or become miscible with the crosslinking agent and swell the particles of crosslinked polymer. A mixture of two or more such liquid polymerizable monomers may be used.

MONOFUNCTIONAL MONOMERS FOR PREPARATION OF CROSSLINKED POLYMER FILLERS

Monomers useful in the production of the crosslinked polymers used in the practice of preferred embodiments of the invention include methyl-, ethyl-, isopropyl-,tert-butyloctyl-, dodecyl-, cyclohexyl-, chloromethyl-, tetrachloroethyl-, perfluorooctyl-, hydroxyethyl-, hydroxypropyl-, hydroxybutyl-, 3-hydroxy-phenyl-, 4-hydroxyphenyl-, aminoethyl-, aminophenyl-, and thiophenyl-, acrylate, methacrylate, ethacrylate, propacrylate, butacrylate and chloromethacrylate, as well as the homologous mono-acrylic acid esters of bisphenol-A, dihydroxydiphenyl sulfone, dihydroxydiphenyl ether, dihydroxybiphenyl, dihydroxydiphenyl sulfoxide, and 2,2 bis(4-hydroxy-2,3,5,6-tetrafluorophenyl)propane. Other suitable species will be apparent to those skilled in the art.

POLYFUNCTIONAL MONOMERS FOR PREPARATION OF CROSSLINKED POLYMER FILLERS

Crosslinking agents which are useful in the production of the crosslinked polymer component of preferred embodiment of the invention include a wide variety of di- or polyfunctional moieties which are capable of crosslinking monomer species, for example, acrylic and lower alkyl acrylic acid diesters, acrylic and lower alkyl acrylic acid esters formed from alcohols, which alcohols have a second reactive function, urethane diacrylates and dimethacrylates, polyvinylic compounds, divinyl aromatic compounds, esters of unsaturated acids, e.g., acrylic, methacrylic, ethacrylic, propacrylic, butacrylic, etc., maleic, fumaric, citraconic, mesaconic, itaconic, malonic, or aconitic, etc., acids preferably reacted with either unsaturated or polyhydroxylic alcohols to form esters which are effective polyfunctional crosslinking agents useful in the formulation of the crosslinked polymers of the invention. In general, these alcohols have one or more hydroxylic functionalities and have from 2 to about 30 carbon atoms. Thus, useful alcohols include allyl, methallyl, crotyl, vinyl, butenyl, isobutenyl and similar unsaturated alcohols as well as polyols such as ethylene glycol, propylene glycol, butylene glycol, diethylene glycol, triethylene glycol, tetraethylene glycol, pentaethylene glycol, glycerol, 1,3,3-trimethylolpropane, pentaerythritol, dihydroxyphenol, and alkylidene bisphenols such as bisphenol-A, 1,1-bis(4-hydroxy-phenyl)-methane, 4,4'dihydroxybiphenyl, 4,4'-dihydroxydiphenyl sulfone, dihydroxydiphenyl ether, dihydorxydiphenyl sulfoxide, resorcinol, hydroquinone, etc. and esters of a mono- or dibasic unsaturated acid with an unsaturated monohydroxylic alcohol such as allyl acrylate, allyl methacrylate, vinyl acrylate (methacrylate and $C_1$ to $C_{20}$ homologs), dimethallyl fumarate, N-allyl acrylamide, crotyl acrylate, allyl crotonate, allyl cinnamate, diallyl maleate, etc. di-, tri-, and higher esters of polyhydroxylic alcohols such as ethylene "glycol" diacrylate (dimethacrylate and $C_2$-$C_{40}$ homologs), trimethylolpropane trimethacrylate, and the diacrylate and dimethacrylate esters of bisphenol-A as well, as acrylate and alkyl acrylate esters which correspond to the general formula

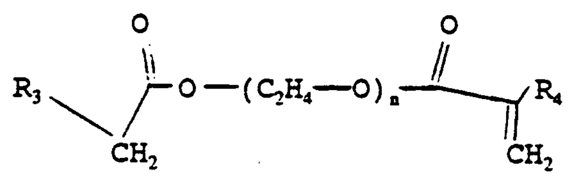

where $R_3$ and $R_4$ may be the same or different and are hydrogen or alkyl groups containing from 1 to about 6 carbon atoms and n is a whole number from 1 to about 10. Alternatively, the crosslinking agent may conform to the formula

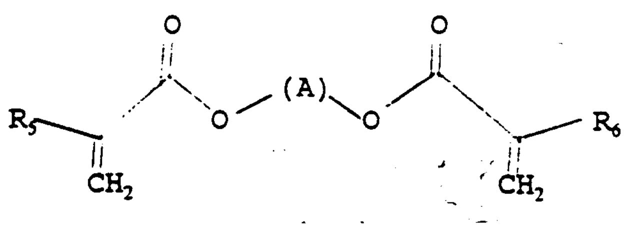

where $R_5$ and $R_6$ may be the same or different and are hydrogen or alkyl groups containing from 1 to about 6 carbon atoms and A is an aromatic moiety selected from the group consisting of (a) biphenyl, diphenyl alkylidene having from 1 to about 6 carbon atoms in the alkylidene portion thereof, diphenyl sulfone, diphenyl sulfoxide, diphenyl ether, and diphenyl sulfide; (b) the diglycidyl derivatives of group (a); and (c) the diurethane derivatives of either group (a) or group (b). In addition, the crosslinking agent may be a glycidyl acrylate or allyl acrylate, divinyl (trivinyl or higher homologs) benzene, substituted divinyl benzenes, and analogous compounds. Furthermore, mixtures of two or more crosslinking agents are useful in the practice of the invention.

Compounds such as bis-GMA and the urethane diacrylate formed by reacting hydroxyethyl acrylate hydroxypropyl acrylate and their methacrylic homologs with 2,4,4-trimethylhexyl-1,6-diisocyanate are especially useful, as are diallyl maleate, ethylene "glycol" dimethacrylate, trimethylolpropane trimethacrylate and the dimethacrylate ester of bisphenol-A.

CROSSLINKED POLYMER FILLERS

Crosslinked polymer which may be prepared from the ingredients above or others which are useful in the practice of the preferred interpenetrating network embodiment of the invention are formed from monomers or blends of monomers together with crosslinking agents. The monomers suitable for use in the production of the crosslinked polymers

include acrylic and lower alkyl acrylic acid esters, N-vinyl lactams, acrylamides, acrylonitriles, styrenes, alkenes, and urethanes. Preferred monomeric species useful in the preparation of the composition of the invention include acrylic and lower alkyl acrylic acid esters which generally conform to the structure:

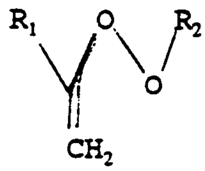

wherein $R_1$ is hydrogen or an alkyl group including from 1 to about 6 carbon atoms, and where $R_2$ is either (a) an alkyl or cycloalkyl group including from 1 to about 20, and preferably from 1 to about 6 carbon atoms; (b) phenyl and (c) alkyl substituted phenyl in which the alkyl groups include form 1 to about 6 carbon atoms. Various substituents may be present on either or both of the groups $R_1$ and $R_2$. Thus, hydroxyl, carboxyl, amino, thiol and halogen (e.g., fluorine, chlorine, etc.) functionalities may be present, with the latter being preferred. Fluorine is an especially suitable and useful substituent.

The crosslinked polymer fillers are produced by polymerizing a mixture of the monomer or monomers and crosslinking agent or agents described above. The amount of crosslinking agent employed in the production of the crosslinked polymers used in the practice of the invention is a critical factor. It has been found that the capacity of particles of polymers so produced to swell with or to imbibe the liquid components forming the precursor blend of the invention, is directly related to the amount of crosslinking agent used in the production of such crosslinked polymers.

The physiochemical properties of the crosslinked polymer fillers useful in the preferred interpenetrating network embodiment of the invention determine the relative proportions of monomer and crosslinking agent used to formulate said suitable crosslinked polymers. Such crosslinked polymers must be sufficiently well crosslinked as to maintain substantially their structural identity when exposed to the liquid components of the precursor blend of the invention. At the same time, they must not be so thoroughly crosslinked as to be incapable of swelling with or imbibing such liquid components. Thus, it is convenient to describe the proportion of crosslinking agent by what it does rather than by what it is. In view of the fact that the crosslinked polymers are utilized in finely particulate form, as will be more fully explained, it is convenient to define the minimum amount of crosslinking agent used therein as being that amount which is sufficient to cause the particulate crosslinked polymer not to lose its particulate discreteness upon exposure to the liquid components of the invention. Similarly, the maximum amount of crosslinking agent used therein is that amount beyond which the resulting crosslinked polymer particles are unable to swell with or further imbibe a significant portion of liquid components upon exposure thereto. In this regard, a quantity of crosslinked polymer particles would be said to swell with or imbibe a significant portion of liquid components if it swelled with or has imbibed at least 10% of its own weight of such liquid. Preferably, an amount of crosslinking agent is used to provide a crosslinked polymer having the capacity to imbibe from about 10 to about 500 percent of its own weight of liquid components.

It will be clear to those skilled in the art that the minimum and maximum values for the proportions of crosslinking agents suitable for inclusion in the crosslinked polymers of this invention will vary depending upon the chemical identity of the component monomers and crosslinking agents. In general, however, the crosslinking agents may comprise from as low as about 0.01% to as high as about 100 and preferably from about 0.2% to about 40 by weight of the resulting crosslinked polymer.

The production of the crosslinked polymer fillers useful in the preferred interpenetrating network embodiment of the invention from monomers and crosslinking agents may be performed by any of the many processes known to those skilled in the art. Thus, the polymers may be formed by heating a mixture of the components to a temperature sufficient to cause polymerization, either with or without the addition of initiators. For this purpose, peroxy type initiators such as benzoyl peroxide, dicumyl peroxide and other materials familiar to those skilled in the art may be employed and the use of activators may be advantageous in some formulations. Alternatively, the crosslinked polymers of the invention may be formed from the constituents by photochemical or radiant initiation utilizing light or high energy radiation.

The polymerization of the crosslinked polymers may be accomplished in a wide variety of ways all of which are known to those skilled in the art. Thus, they may be formed by suspension polymerization (as taught Grim in U.S. Patent No. 2,673,194), emulsion polymerization, block polymerization. The crosslinked particles preferably have an average particle size should be from about 0.001 micron to about 500 microns. It is preferred that at least 50% by weight of the particles have diameters below 150 microns and more preferably below 100 microns.

UNCROSSLINKED POLYMER FILLERS

In addition to the crosslinked polymers described above, the polymer component of the precursor blend may comprise an uncrosslinked polymer. Such uncrosslinked polymer include those formed from any of the monofunctional monomer species which have been disclosed above as being useful for the preparation of the crosslinked polymers used in the practice of the invention. Thus, monomer species conforming to the formula above, the acrylic and $C_1$ to $C_6$ lower alkyl acrylic esters of aliphatic alcohols of phenols having from 1 to about 20 carbon atoms, or mixtures thereof, are suitable as a vinylidene fluoride. Polymeric methyl methacrylate and copolymers thereof are preferred. The uncrosslinked polymers may be formed from the monomers through any of the polymerization procedures known to those skilled in the art. Thus, thermal or photochemical polymerization, either with or without initiators, sensitizers, activators or chain transfer agents may be employed. Similarly, either bulk or suspension polymerization may be utilized. Preferably, the uncrosslinked polymers should be characterized as having average molecular weight of from about 100,000 to about 2,000,000 g/mole, and especially of from about 500,000 to about 900,000 g/mole. While the polymers are used in particulate form, they differ from the crosslinked polymer filler in that, unlike the crosslinked polymers, the uncrosslinked polymers do not have a critical particle size distribution. Thus, polymer particles or beads of any conveniently small size such as about 50 microns, may be utilized. Smaller sizes are preferred since they imbibe monomers and will dissolve therein more readily, but larger sizes may be used as well.

POLYMERIZABLE LIQUID BLEND

Polymerizable monomers suitable for use in the formulation of the precursor blend of the invention may comprise any of a wide variety of monomers. Thus, acrylic and lower alkyl acrylic acid esters, N-vinyl lactams, acrylimides, acrylamides, acrylonitriles, styrenes, alkenes, urethane acrylate or methacrylate and other monomeric species may be employed in the practice of the invention.

Especially preferred examples of polymerizable monomers useful in the practice a preferred embodiment of the invention include methyl-, ethyl-, isopropyl-, t-butyl-, octyl-, dodecyl-, cyclohexyl-, chloromethyl-, tetrachloroethyl-, perfluorooctyl-, hydroxyphenyl-, hydroxypropyl-, hydroxybutyl-, 3-hydroxyphenyl-, 4-hydroxyphenyl-, aminoethyl-, aminophenyl-, and thiophenyl-, acrylate, methacrylate, ethacrylate, propacrylate, butacrylate and chloromethacrylate, as well as the homologous mono-acrylic acid esters of bisphenol-A, dihydroxydiphenyl sulfone, dihydroxydiphenyl ether, dihydroxybiphenyl, dihydorxydiphenyl sulfoxide, and 2,2-bis(4-hydroxy-2,3,5,6-tetrafluorophenyl)propane. Other suitable species will be apparent to those skilled in the art who will further recognize that mixtures of two or more different polymerizable monomers may be used.

POLYFUNCTIONAL MONOMERS AND OLIGOMER COMPONENTS OF POLYMERIZABLE LIQUID BLEND

Preferably, the crosslinking agents for the polymerizable monomers comprise esters of unsaturated acids, e.g., acrylic, methacrylic, ethacrylic, propacrylic, butacrylic, etc. maleic, fumaric, citraconic, mesaconic, itaconic, malonic, or aconitic, etc., acids. Other unsaturated acids will be readily apparent to those skilled in the art. These acids are preferably reacted with either unsaturated or polyhydroxylic alcohols to form esters which are effective polyfunctional crosslinking agents for the monomeric species useful in the practice of the invention. Thus, useful alcohols include allyl, methallyl, crotyl, vinyl, butenyl, isobutenyl and similar unsaturated alcohols as well as polyols such as ethylene glycol, propylene glycol, butylene glycol, diethylene glycol, triethylene glycol, tetraethylene glycol, pentaethylene glycol, glycerol, trimethylolpropane, pentaerythritol, dihydroxyphenol, alkylidene bisphenols such as bisphenol-A; 1,1-bis(4-hydroxyphenyl)methane; 4,4'-dihydroxybiphenyl; 4,4'-dihydroxydiphenyl sulfone; dihydroxydiphenyl ether; dihydroxydiphenyl sulfoxide; resorcinol; hydroquinone; etc.

The preferred crosslinking agent used in the practice of the invention include the esters of a monomeric dibasic unsaturated acid with an unsaturated monohydroxylic alcohol such as allyl acrylate, allyl methacrylate, vinyl acrylate (methacrylate and homologs), dimethallyl fumarate, N-allyl acrylamide, crotyl acrylate, allyl crotonate, allyl cinnamate, diallyl maleate, etc. Other preferred species are the di-, tri-, and higher esters of polyhydroxylic alcohols such as ethylene "glycol" diacrylate (dimethacrylate and $C_2$-$C_6$ homologs), trimethlolpropane trimethacrylate, and the dimethacrylate ester of bisphenol-A as well as other acrylate and allyl acrylate esters. In addition, the crosslinking agent for the polymerizable monomers may be a glycidyl acrylate or allyl acrylate, divinyl (trivinyl or higher homologs) benzene, substituted divinyl benzenes, or analogous compounds. Furthermore, mixtures of crosslinking agents are useful in the practice of the invention.

Compounds such as bis-GMA and the urethane dimethacrylate formed from the reaction of hydroxyethyl acrylate, hydroxypropyl acrylate and their methacrylate homologs with 2,4,4-trimethylhexyl-1,6-diisocyanate (hereinafter referred to as "urethane dimethacrylate" or "diacrylate") are especially useful, as are ethylene "glycol" dimethacrylate, trimethylolpropane trimethacrylate and the dimethacrylate ester of bisphenol-A. The corresponding acrylates are similarly useful as is diallyl maleate.

ADDITIONAL INGREDIENTS

In addition to the components described above, (i.e., crosslinked polymer, uncrosslinked polymer, polymerizable monomer) the precursor blend may contain additional, optional, ingredients, such as, initiators, activators, pigments, fillers, radiopaquing agents, adhesion modifiers, free radical or photochemical initiators. In this regard, peroxy type initiators such as dicumyl or benzoyl peroxide are useful. Similarly, pigments and fillers may be added to modify the appearance, density, and physical characteristics of the resultant dental appliances. Inorganic materials, especially silica and titania, are useful fillers and pigments while a wide variety of other useful pigments and fillers will be apparent to those skilled in the art. The fillers and radiopaquing agents may constitute a major part by weight of the compositions of the invention. According to a preferred embodiment, the precursor blend of this invention may comprise admixtures of organic resin components and particulate, inorganic filler in weight ratios of from about 1:2 to about 2:1.

PRECURSOR BLENDS

The precursor blends in accordance with a preferred embodiment of the invention are formulated by a mixing together of the constituent species in proper proportion, followed by aging or maturing. Several techniques are available for this and others will be apparent to those skilled in the art. Thus, it is possible to combine crosslinked polymer filler, self-lubricating particles, uncrosslinked polymer and polymerizable liquid blend in proper proportions including therewith, for example, a peroxide initiator and a pigment. This combination is then thoroughly mixed and aged to result in a precursor blend which has a uniform appearance. This blend may have the consistency of dough or may be more or less mobile depending upon the desired use thereof. Particulate inorganic fillers or other modificants may be preferably added at this stage in the formulation of the compositions if desired. The compositions thus formed may be alternatively molded, extruded, brushed, formed, worked or otherwise shaped in any conventional manner and caused to polymerize or cure to result in hard dental appliances having superior properties. The application of heat or radiant energy is usually required for this polymerization or curing.

PROCEDURE

It is especially useful to mold the compositions of this invention into artificial teeth for inclusion in prosthetic devices. It is to be understood, however, that the precursor blends are suitable for a very wide range of dental uses, including fillings, teeth, bridges, crowns, veneers, facings, pit and fissure sealants, denture base and denture reline materials, orthodontic splint materials, and dental adhesives. The materials of the invention may also be utilized for prosthetic replacement or repair of various hard body structures such as bone and may be utilized for reconstructive purposes during surgery, especially oral surgery. They are also useful for various non-dental uses as, for example, in plastic construction materials.

The nature of the chemical and physical relationships among the components of the precursor blends of the invention is important to the practice of the invention. Among these relationships is the necessity that the crosslinked polymer particles be capable of swelling with imbibing the liquid components (preferably at least 10% by weight) in compositions for making interpenetrating network in accordance with a preferred embodiment of the invention. Of similar importance is the requirement that the uncrosslinked polymers, when included, be capable of dissolving in the liquid components. The precursor blend formed by any of the useful techniques described above is aged for a period of time sufficient to insure that in one embodiment the crosslinked polymer has become substantially fully swollen with, interpenetrated by or has substantially imbibed the crosslinking agent mixture and that the uncrosslinked polymer, if used, has substantially dissolved therein. Thus, as used herein, "aged" or "aging" refer to the maintenance of the components of the precursor blend in association with one another in the blend for a period of time sufficient to substantially fully swell the crosslinked polymer particles with the mixture of polymerizable monomer and crosslinking agent dissolved therein. Frequently, the aging process is manifested by a change in the consistency of the mixture as equilibrium is approached. The time necessary to approach such equilibrium will vary depending upon the blending techniques, the relative proportions of materials, the particle sizes and molecular weights of the polymers and the temperature extent in the mixtures. In general, aging time of from one to seven days has been found to be adequate to approach the desired equilibrium. It is to be understood that it lies well within the abilities of those skilled in the art to ascertain the optimum aging time for a formulation in view of the foregoing considerations.

A further technique especially useful for the formulation of the precursor blends of the invention, denominated as the preswell method, causes the crosslinked polymer particles to swell with or imbibe a mixture of polymerizable monomer and crosslinking agent for the monomer at a time remote from and preceding the final mixing of the ultimate precursor blend. In accordance with this preferred technique, the crosslinked polymer particles and self-lubricating filler particles are blended with a polymerizable liquid blend. The precursor blend is then aged for a period of time sufficient to permit the crosslinked polymer particles to be substantially fully swollen with, or interpenetrated by polymerizable

blend. Precursor blends thus formed may be alternatively molded, brushed, extruded, formed, worked or otherwise shaped to form useful dental devices and articles. Other techniques are presented in the examples which follow, and still others will be apparent to those skilled in the art.

Upon polymerization of the precursor blends in one embodiment a three dimensional structure is believed to be formed which may be denominated as an interpenetrating polymeric network. The interpenetrating network structure which is believed to form is a major contributing factor to the serendipitous combination of superior chemical and physiochemical properties which is exhibited by the articles constructed according to the practice of the invention. Interpenetrating polymeric networks are related to, but distinct from, traditional graft polymers.

INTERPENETRATING NETWORK TEETH

In accordance with a preferred embodiment of the invention a self-lubricating interpenetrating network may be viewed as being composed of ultra high molecular weight polyethylene and two or more crosslinked, and hence three dimensionally arrayed, polymeric networks which co-exist in the same volume of space, but which do not necessarily have any covalent bonds in common. While the two networks may, indeed, be independent in the sense that they need possess no covalent linkages between them; they are physically trapped one "within" the other and cannot disassociate by any physical manipulation without the rupture of covalent bonds. Particulate crosslinked polymer is allowed to swell with or imbibe monomer mixed with crosslinking agent, and when the imbibed mixture of monomer and crosslinking agent is subsequently caused to polymerize, an interpenetrating polymeric network may be seen to be formed within the confines of the particulate crosslinked polymer. It is believed that the aging process employed in the preparation of the precursor blends of the invention is required to accomplish substantially full swelling with interpenetration by or substantially complete inbibition of crosslinking agent by the crosslinked polymer particles, and to approach an equilibrium thereof.

The American Dental Association specification number 15 specifies, "the strength of the bond between tooth and resin is tested in tension. The minimum bond strength is 30.9 $MN/M^2$(4,480 psi; 315 $Kg/cm^2$), which is sufficient to prevent separation of the teeth from the resin denture base in use." This pertains to "acrylic denture base resin polymerized by the heat processing technique." The compositions of this invention meet or exceed this specification.

A unique, heterogeneous microstructure is exhibited by one embodiment, the preferred interpenetrating network embodiment of the invention. One exemplary method for observing this microstructure is as follows:

1. The tooth, or molded article, is sectioned and one section potted in epoxy against a flat surface.
2. The sectioned surface of the potted specimen is polished to a smooth surface using nos. 320, 400 and 600 grit silicon carbide papers wet continuously with water.
3. A final polish is obtained using an aqueous slurry of 0.3 micron $Al_2O_3$ on a chamais.
4. The polished surface of the section is exposed for four minutes to the vapors of boiling concentrated nitric acid; the microstructure is oxidatively exposed by this etching procedure and is best captured by photomicrography at 260 x magnification.

The microstructure thus observed is heterogeneous and comprises what may best be described as particles suspended in a matrix. The particles are believed to be identifiable with the particulate crosslinked polymers of the precursor blend which have been swollen by and interpenetrated with the monomer and crosslinking agent. By comparison with conventional composite compositions containing only rigid inorganic fillers, the articles formed according to the present invention exhibit a microstructure in which the structure is much more closely packed. It is to be understood that this methodology, while of wide application in the examination of the microstructure of the novel compositions of the invention, is not exclusive. Other techniques involving greater or lesser magnification and other means of visualization are also useful in disclosing the structure.

Preferably teeth formed in accordance with a preferred embodiment of the invention are from 0.1 to 80 percent by weight ultra high molecular weight polyethylene and have an outer surface having a kinetic coefficient of friction less than 0.2 when measured using ASTM D 1894-78 and 5 psi. More preferably such teeth are from 2 to 50 percent by weight ultra high molecular weight polyethylene, and have an outer surface having a kinetic coefficient of friction less than 0.1 when measured using ASTM D 1894-78 and 5 psi. Most preferably such teeth are from 1 to 10 percent by weight ultra high molecular weight polyethylene and have a kinetic coefficient of friction less than 0.05 when measured using ASTM D 1894-78 and 5 psi.

Preferably teeth formed in accordance with a preferred embodiment of the invention are from 0.05 to 50 percent by weight silicon containing self-lubricating compound. More preferably such teeth are from 0.5 to 20 percent by weight of a silicon containing self-lubricating compound. Most preferably such teeth are from 1 to 10 percent by weight silicon containing self-lubricating compound. Most preferably the abrasion resistant monomeric silicone is divinyldimethyl silane or divinyldimethyl siloxane.

Preferably restorative material formed in accordance with a preferred embodiment of the invention are from 0.1 to 50 percent by weight ultra high molecular weight polyethylene. More preferably such restorative material is from 2 to 20 percent by weight ultra high molecular weight polyethylene. Most preferably such restorative material is from 1 to 10 percent by weight ultra high molecular weight polyethylene.

Preferably, the high molecular weight polyethylene particles for use in accordance with the invention have an average largest dimension (or diameter) from 0.5 to 80 microns. More preferably, such particles have an average largest dimension (or diameter) less than 70 microns. Most preferably, the high molecular weight polyethylene particles for use in accordance with the invention have an average largest dimension (or diameter) from 1 to 80 microns. Most preferably, the high molecular weight polyethylene particles for use in accordance with the invention have an average largest dimension (or diameter) from 2 to 20 microns. A preferred embodiment of the invention provides a tooth including polyethylene having a molecular weight of at least 500,000 and preferably a molecular weight of at least 1,000,000, and a particle size less than 80 microns. In a preferred embodiment of the invention a dental restorative resin is provided having polyethylene particles having a particle size less than 80 microns and a molecular weight of at least 3,000,000.

Preferably restorative material formed in accordance with a preferred embodiment of the invention are from 0.02 to 80 percent by weight silicon containing self-lubricating compound. More preferably such restorative material is from 0.5 to 40 percent by weight silicon containing self-lubricating compound. Most preferably such restorative material is from 1 to 10 percent by weight silicon containing self-lubricating compound.

The following examples describe certain representative embodiments of this invention and will serve further to illustrate the nature thereof. It is to be understood that the examples are merely illustrative, and do not in any way limit the scope of the invention as defined by the claims. All percentages are by weight.

The composition of the highly wear resistant polymeric tooth material is made from an interpenetrating methacrylated polymer network with covalently bonded ultrahigh molecular weight polyethylene particles.

EXAMPLE 1A

TOOTH FILLING MATERIAL

20 g ultrahigh molecular weight polyethylene particles with a largest dimension less than 80 microns and treated with oxygen and fluorine from Air Products (Primax); 61.5 g of microfine silica filler (average particle size between 10 and 20 mμ with a BET surface area of 50 $m^2/g$) are silanized in the customary manner with methacryloxy-propyltrimethoxy silane and poured into a laboratory kneader. 22 g of Bis-GMA and 16.5 g of ethyleneglycol-dimethacrylate are added thereto and the kneading is continued until a homogeneous paste which is free of specks is obtained. 40 g of this paste are treated with 0.6 g of 50% benzoylperoxide (paste A). A further 40 g of the master paste are treated with 0.1 g dimethylpara-toluidine (paste B).

Equal quantities of paste A and B are mixed on a mixing block to obtain a filling material for dental cavities. The working time is 2 minutes; by 5 minutes the material is hard.

EXAMPLE 1B

ACRYLIC TOOTH

100 g of the micro-fine filler and UHMWPE in the ratio used in Example 1A but which was not silanized are mixed with 30 g of uncolored polymethylmethacrylate present in the form of bead polymer as well as 2 g of 50% benzoylperoxide. A monomer mixture is prepared consisting of 35 g of monomeric methylmethacrylate and 35 g of a reaction product of hydroxyethyldimethacrylate and hexamethylene-diisocyanate. The powder and the liquid are mechanically mixed in a closed container on a swing mixing device until a viscous paste is obtained. This paste is introduced into a tooth mold and polymerized for 4 minutes at 110°C. The artificial tooth thus obtained shows clear opalescence, i.e. it appears yellowish in transmitted light and of a blue-white transparency in incident light. The ball indentation hardness is 2800 $kg/cm^2$ as compared with 1400 $kg/cm^2$ in the case of comparison teeth compared on basis of the customary methacrylate. The resistance of the plastic tooth containing the microfine filler to monomer, chloroform or boiling water is definitely better.

PROSTHETIC TEETH

EXAMPLE 2A

PRECURSOR BLEND

A precursor blend is prepared from the following composition:

| | |
|---|---|
| 45.83% | methyl methacrylate |
| 0.17% | benzoyl peroxide |
| 11.50% | 2,2-bis(4-methacryloxyphenyl)propane |
| 24.80% | poly(methyl methacrylate-co-ethylene dimethacrylate) (98.4:1:6) |
| 11.90% | poly(methyl methacrylate) |
| 1.80% | pigment |
| 100.00% | |

The crosslinked polymer is in the form of particles, 46% by weight of which were below 74 microns in size, the balance being below about 500 microns in size. The poly(methyl methacrylate) have an average molecular weight of 800,000 g/mole.

The benzoyl peroxide and 2,2-bis(4-methacryloxyphenyl)propane are dissolved in the methyl methacrylate at ambient temperature to form a monomer solution. The polymers and pigment are charged to a planetary dough mixer containing the monomer solution and the charge is stirred until visibly homogeneous. Prosthetic teeth are molded from the resultant precursor blend mixture after it is aged at ambient temperature for seven days. The resulting teeth grind with a dusty, fine debris, bond to denture base and are impact and wear resistant.

EXAMPLE 3A

INTERPENETRATING NETWORK TEETH

The method described in Example 2A is used to prepare a precursor blend from which prosthetic teeth are molded having the following composition:

| | |
|---|---|
| 26.83% | methyl methacrylate |
| 4.00% | ultrahigh molecular weight polyethylene treated with oxygen and fluorine (50 micron largest particle dimension |
| .17% | benzoyl peroxide |
| 2.11% | 2,2,2-trifluoroethyl acrylate |
| 2.37% | ethylene "glycol" dimethacrylate |
| 1.52% | urethane diacrylate |
| 41.30% | poly(methyl methacrylate-co-ethylene dimethacrylate) (98.8:1.2) |
| 20.65% | poly(methyl methacrylate) |
| 1.05% | pigment |
| 100.00% | |

A suitable gel-like consistency for molding proshetic teeth is obtained after aging at ambient temperature for 24 hours.

EXAMPLE 4A

INTERPENETRATING TEETH

The following composition yielded a precursor blend which is molded into prosthetic teeth after processing according to the technique of Example 3A:

| | |
|---|---|
| 45.83% | methyl methacrylate |
| 4.00% | ultrahigh molecular weight polyethylene treated with oxygen and fluorine (50 micron largest particle dimension |
| 0.17% | benzoyl peroxide |

| | |
|---|---|
| 11.50% | bis-GMA |
| 24.80% | poly(methyl methacrylate-co-ethylene dimethacrylate) (70:30) |
| 11.90% | poly(methyl methacrylate) |
| 1.80% | pigment |
| 100.00% | |

EXAMPLE 5A

INTERPENETRATING NETWORK TEETH

A two-step "preswell" mixing method is used to prepare a precursor blend from which prosthetic teeth are molded. The blend has the following composition:

Step 1

| | |
|---|---|
| 40.40% | methyl methacrylate |
| 4.00% | ultrahigh molecular weight polyethylene treated with oxygen and fluorine (50 micron largest particle dimension) |
| 0.25% | benzoyl peroxide |
| 6.00% | urethane diacrylate |
| 1.50% | 2,2-bis(4-methacryloxyphenyl)propane |
| 47.85% | poly(methyl methacrylate-co-ethylene dimethacrylate) (90:10) |
| 100.00% | |

The crosslinked polymer is in the form of particles, 50% by weight of which are below 100 microns in size, the balance being below about 500 microns in size.

Step 2

| | |
|---|---|
| 28.14% | poly(methyl methacrylate) |
| 60.43% | methyl methacrylate |
| 0.36% | benzoyl peroxide |
| 10.20% | 2,2-bis(4-methacryloxyphenyl)propane |
| 0.87% | pigment |
| 100.00% | |

The poly(methyl methacrylate) has an average molecular weight of 850,000 g/mole.

The weight ratio of Step 1 to Step 2 material in this example is 1.14 to 1.00. Step 1 is achieved by preparing a solution of the monomers, crosslinkers and initiator and adding the crosslinked copolymer. This mixture is stirred for about two minutes to wet the polymer, capped against monomer loss, and held for one week at about 10°C temperature. The crosslinked copolymer completely absorbed the monomer solution during the one week "pre-swell" period. Although the copolymer is swollen by this process, the integrity of the individual copolymer particles is maintained. This "preswell" mixture is not gel-like, but has the consistency of a rubbery, spongy mass which is easily crumbled.

Step 2 is achieved by charging "preswell", obtained in Step 1, to a planetary dough mixer and mixing sufficiently so as to break the "preswell" mass of own to a fine consistency. The poly(methyl methacrylate) and pigment are added to the mixer and mixing is continued until a homogeneous dispersion is obtained. The solution of monomer and initiator, cited in the Step 2 composition, is charged to the mixer; mixing continued until a homogeneous, gel consistency is obtained. The gel-like mix is transferred to a holding container and aged at about 10°C temperature until a suitable consistency for molding prosthetic teeth is obtained, approximately three days.

EXAMPLE 6A

INTERPENETRATING NETWORK TEETH

The two-step "preswell" method described in Example 6A is used to prepare a precursor blend from which prosthetic teeth are molded having the following composition:

Step 1

| | |
|---|---|
| 39.90% | methyl methacrylate |
| 0.24% | benzoyl peroxide |
| 9.98% | 2,2-bis(4-methacryloxyethyoxyphenyl)propane |
| 49.88% | poly(methyl methacrylate-co-ethylene dimethacrylate) (98:2) |
| 100.00% | |

Step 2

| | |
|---|---|
| 35.37% | poly(methyl methacrylate) |
| 3.00% | ultrahigh molecular weight polyethylene treated with oxygen and fluorine (50 micron largest particle dimension) |
| 47.92% | methyl methacrylate |
| .32% | benzoyl peroxide |
| 12.19% | 2,2-bis(4-methacryloxyethoxyphenyl)propane |
| 1.20% | pigment |
| 100.00% | |

The weight ratio of Step 1 to Step 2 material in this example is 0.46 to 1.00. A suitable gel-like consistency for molding prosthetic teeth is obtained after aging at ambient temperature for 24 hours.

EXAMPLE 7A

ANTERIOR PROSTHETIC TEETH

A precursor blend was prepared from the following composition:

| | |
|---|---|
| 24.17% | methyl methacrylate |
| 3.00% | ultrahigh molecular weight polyethylene treated with oxygen and fluorine (50 micron largest particle dimension) |
| 0.25% | benzoyl peroxide |
| 24.17% | ethylene "glycol" dimethacrylate |
| 47.70% | poly(methyl methacrylate-co-ethylene dimethacrylate) (99.8:0.2) |
| 0.71% | pigment |
| 100.00% | |

The methyl methacrylate, benzoyl peroxide, and ethylene "glycol" dimethacrylate are mixed at ambient temperature to form a monomer solution. The polymer and pigment are charged to a planetary dough mixer containing the monomer solution and then mixed until visibly homogeneous. The polymer completely imbibed the monomer solution during the first seven days of contact at ambient temperature in a sealed container; aging is continued for seven days of contact at about 10°C temperature in a sealed container; aging is continued for seven days prior to molding. Monolithic anterior prosthetic teeth are transfer molded by the following sequence:

1. 3 min. at 138°C, 290 psi.
2. 2 min. at 138°C, 1300 psi.
3. 5 min. cool at 1300 psi.
4. 3 hr. at 118°C.

The resultant prosthetic teeth grind with a fine dusty debris, repolish to a high gloss, resist wear, resist methyl methacrylate and other solvents, are hydrolytically stable, show no visible degradation or distortion when heated at 220°C for one hour, and bond well to denture base material.

ONE-COMPONENT FILLED RADIATION

CURABLE MATERIAL

EXAMPLE 8A

The following gel composition, containing an inorganic filler and prepared by the method described in Example 3A, is polymerized by ultra violet radiation having wavelengths of 320 to 400 nanometers using a Caulk NUVA-LITE photocure (registered trademark of Dentsply International):

| | |
|---|---|
| 21.21% | methyl methacrylate |
| 3.00% | ultrahigh molecular weight polyethylene treated with oxygen and fluorine (50 micron largest particle dimension) |
| 2.96% | butyl methacrylate |
| 0.27% | 2,2-diethoxyacetophenone |
| 0.43% | 2,2-dimethoxy-2-phenylacetophenone |
| 2.08% | 2,2-bis(4-methacryloxyethoxyphenyl)-propane |
| 1.13% | tetraethylene "glycol" dimethacrylate |
| 1.13% | neopentyl "glycol" dimethacrylate |
| 19.45% | poly(methyl methacrylate-co-2,2-bis-(4-methacryloxyphenyl)propane) (99.8:0.2) |
| 12.23% | poly(methyl methacrylate) |
| 35.66% | silane treated, fine (12 micron) particle quartz |
| 0.45% | pigment |
| 100.00% | |

STABLE ONE PART DENTAL VENEERS

EXAMPLE 9A

A two step preswell process is used to mix a one part dental veneer material. A blend is prepared from the following:

Step 1

| | |
|---|---|
| 2.99% | methyl methacrylate |
| 3.00% | ultrahigh molecular weight polyethylene treated with oxygen and fluorine (50 micron largest particle dimension) |
| 0.51% | benzoyl peroxide |
| 45.26% | reaction product of hydroxypropyl methacrylate with 2,2,4-trimethylhexyl-1,6-disocyanate(2:1) (methane urethane dimethacrylate) |
| 48.24% | poly(methyl methacrylate-co-ethylene DIMETHACRYLATE) (99.8:0.2) |
| 100.00% | |

The benzoyl peroxide is dissolved in the methyl methacrylate and blended with the urethane dimethacrylate. This solution is then mixed with the poly(methyl methacrylate-co-ethylene dimethacrylate) (99.8:0.2) and stored in the dark in a sealed jar to become the "preswell" blend. The crosslinked polymer is in the form of fine particles at least 50% by weight are below 100 microns in size, and the balance below 500 microns in size. After one month storage the fully swollen crosslinked polymer "preswell" blend is admixed as follows:

Step 2

| | |
|---|---|
| 48.84% | "preswell" blend from Step 1 |
| 51.03% | silane treated microfine silica |
| 0.13% | acrylic acid |
| 100.00% | |

These components are mixed on a three roll mill with minor amounts of pigments as required until a uniformly shaded paste is obtained.

A veneer is prepared on an opaqued crown by the well known build up method. A dentin shade veneer paste is built up on the crown by hand and instrument modelling. Next, an incisor shade veneer paste is built on top of the dentin. The veneer is polymerized by immersion in a 90°C water bath under three bars air pressure. Veneers are also polymerized by immersion in glycerin in a similar manner. The finished veneer has a high gloss and good aesthetic appearance. The veneer has three times the wear resistance of conventional acrylic veneers by a prophy abrasion test. The veneer can be readily shaped by grinding, yielding a dusty debris, and then is readily polished to a smooth, high gloss finish. The veneer is resistant to chemicals and stains, has good impact strength and is repairable. The veneer paste is stable at ambient conditions. The veneer paste is stable for nine months at ambient and seventy days at 50°C.

EXAMPLE 10A

DENTAL VENEER

A two step preswell process is used to mix a one part dental veneer from the following composition:

Step 1

"Preswell" blend from Example 10 aged 22 days at ambient.

Step 2

| | |
|---|---|
| 57.29% | "preswell" blend from Step 1 |
| 41.67% | silane treated microfine silica |
| 1.04% | acrylic acid |
| 100.00% | |

The components are mixed on a three roll mill until visually uniform. The putty-like material is formed into a dental veneer and polymerized by immersion in 90°C glycerin at three bars pressure for ten minutes to yield shapable, durable and aesthetically superior appliances.

EXAMPLE 11A

TEETH INCLUDING UHMWPE

Polyethylene treated with oxygen and fluorine having a molecular weight of about 4,000,000 from Air Products (Primax) is molded into teeth in a mold at 160 degrees C. The teeth are allowed to cool to 23 degrees C and removed from the mold.

EXAMPLE 12A

FILLING MATERIAL INCLUDING ULTRA HIGH MOLECULAR WEIGHT POLYETHYLENE CONTAINING

60 g of ultra high molecular weight polyethylene particles having a largest dimension less than 80 microns and a molecular weight of at least 1,000,000 is added to 61.5 g of silica (average particle size between 10 and 20 mu and with a BET surface area of 50 $m^2/g$) are silanized in the customary manner with methacryloxypropyltrimethoxy silane and poured into a laboratory kneader. 22 g of Bis-GMA and 16.5 g of ethyleneglycoldimethacrylate are added thereto and the kneading is continue until a homogeneous paste which is free of specks is obtained. 40 g of this paste is treated with 0.6 g of 50% benzoylperoxide (paste A). A further 40 g of the master paste is treated with 0.1 g of dimethylpara-toluidine (paste B).

Equal quantities of pastes A and B are mixed on a mixing block to obtain a filling material for dental cavities. The working time is 2 minutes; by 5 minutes the material is hard.

EXAMPLE 13A

CROWN INCLUDING SILICON CONTAINING SELF-LUBRICATING COMPOUND

20 g of 2,2-Bis[4-(2-hydroxy-ethoxy)-phenyl]propane dimethacrylate are dissolved in 50 g of chloroform, 21 g of silanized silica having an average particle size of 30 mμ and a BET surface area of less than 80 $m^2/g$ and 5 g of divinyl

dimethyl silane are added to this solution. The paste thus formed is dried with constant stirring until the crystalline monomer is again solid and the solvent has evaporated. In this way the silica is distributed completely homogeneously in the monomer. 0.5% benzoylperoxide is distributed in the powder by grinding it in a ball mill and thereupon screening it. This mixture is stable as long as it is not heated to above 42°C. For processing the powder is melted in a porcelain dish at 50° to 60°C and applied by a brush or spatula to an insulated model stump, it being polymerized layer by layer in a stream of hot air at about 150°C to obtain a crown.

EXAMPLE 14

SELF-LUBRICATING TOOTH MAKING POWDER

Colored tooth making material is prepared by mixing 1 part high molecular weight polymethyl methacrylate polymer from L. D. Caulk (D7-99-WD York PA71); 2 parts poly(methyl methacrylate-co-ethylene glycol dimethacrylate) (99.2:0.8) also called methacrylated copolymer crosslinked with ethylene glycol methacrylate from L. D. Caulk (.8 XL FR2579A Polymer York P448); and 0.01 pigment. Powders are then mixed in an Abbe Ball Mill for 2 hours to form colored tooth making material. Clear tooth making material is prepared by mixing 2 parts of methacrylated co-polymer crosslinked with ethylene glycol methacrylate from L. D. Caulk (.8 XL FR2579A Polymer York P448) which has been ball milled for 2 hours with one part of high molecular weight polymethyl methacrylate polymer from L. D. Caulk (D7-99-WD York P471) in a Papenmeier High Intensity Mixer for 4 minutes to form clear tooth making material. Ultrahigh molecular weight polyethylene (UHMWPE) treated with oxygen and fluorine (Primax from Air Products), is added to the colored tooth making material to form colored self-lubricating tooth making material which is 4% UHMWPE and 96% colored tooth making material. The mixture is tumbled end-over-end in a fiber drum. Ultrahigh molecular weight polyethylene (UHMWPE) treated with oxygen and fluorine (Primax from Air Products), is added to the clear tooth making material to form self-lubricating tooth making material which is 4% UHMWPE and 96% clear tooth making material. The mixture is tumbled end-over-end in a fiber drum. Colored and clear self-lubricating tooth making materials are blended as required to match tooth shade. These powders are tumabled end-over-end in a fiber drum to form blended self-lubricating tooth making powder.

EXAMPLE 2

TOOTH BODIES MATERIALS AND POSTERIOR ENAMEL MATERIAL

657 g methyl methacrylate (a monomer) 138 g Bisphenol-A-Dimethacrylate (a crosslinker), 200 g 2,4,4-trimethyl-1,4-diurethanedimethacrylate hexane (methacrylated end capped) (a second crosslinker) and 5 g Benzoyl Peroxide (initiator) are mixed by adding the initiators and the crosslinkers to the monomer and thoroughly mixing in a large polyethylene carboy for 30 minutes. This resistant liquid is mixed with blended self-lubricating tooth making powder from Example 1 in a ratio of 43% liquid : 57% powder. The liquid and then the powder are added to a 32 pound capacity dough mixer and mixed for 9 minutes. The wet mixture is poured through a spout into polyethylene sleeves which are from 2 and 3/8 inch to 4 and 1/4 inch in diameter. The sleeves are hung vertically until the wet mixture solidifies to a viscosity (determined by a penetrometer reading) between 40 and 50 for tooth enamel material and between 55 and 65 for tooth body material. The sleeves are placed in a freezer for 16 hours and then the tooth material is sliced to 1/8 inch for tooth enamel and 1/4 inch for tooth body. The sliced material is aged for a minimum of 3 days in a freezer and is then molded and heated to 100°C to form tooth bodies and tooth enamels.

EXAMPLE 3

ANTERIOR ENAMEL

Colored and clear tooth making material made as in Example 1 are blended to a desired shade then tumbled end-over-end in a fiber drum to form shaded tooth making material. 795 g methyl methacrylate (a monomer) 175 g Bis-phenol-A-Dimethacrylate (a crosslinker) 25 g divinyldimethylsilane (a second crosslinker) and 5 g Benzoyl Peroxide (initiator), are mixed by adding the initiators and the crosslinkers to the monomer and mixing 30 minutes. This resultant liquid is added to a dough mixer, then the shaded tooth making material is added in a ratio of 46 liquid : 54 shaded tooth making material and mixed for 9 minutes. The wet mixture is poured through a spout into polyethylene sleeves, which are from 2 and 7/8 inch to 4 and 1/4 inch in diameter. The sleeves are hung vertically until wet mixture solidifies to a viscosity (determined by a penetrometer reading) from 40 to 50 for tooth enamel material and 55 to 65 for tooth body material. The sleeves are placed in a freezer for 16 hours and then the wet mixture is sliced to a thickness of 1/8 inch for tooth enamel material and 1/4 inch for tooth body material. The sliced material is aged for a minimum of 3 days in a freezer

and is then molded into tooth bodies and tooth enamels.

EXAMPLE 4

TOOTH CONTAINING UHMWPE

A. A colored powder blend is prepared by mixing 1.0 part by weight of high molecular weight polymethyl methacrylate suspension polymer (D7-99 WD, Dentsply International); 2.0 parts by weight of a slightly crosslinked polymethyl methacrylate suspension polymer, poly (methyl methacrylate-co-ethylene glycol dimethacrylate) (99.2:0), (0.8 XL, Dentsply International); and 0.01 parts by weight pigment. The blend is mixed in an Abbe ball mill for two hours. 0.12 parts by weight ultra high molecular weight polyethylene (UHMWPE) treated with oxygen and fluorine are added to the blend and dispersed by tumbling end-over-end in a fiber drum. The properly shaded blend contains 4% of UHMWPE.

B. An uncolored powder blend is prepared by mixing 1.0 parts by weight of high molecular weight polymethyl methacrylate suspension polymer (D7-99 WD, Dentsply International); 2.0 parts by weight of a slightly crosslinked polymethyl meth-acrylate suspension polymer, poly (methyl methacrylate-co-ethylene glycol dimethacrylate) (99.2:0), (0.8 XL, Dentsply International). The blend is mixed in an Abbe ball mill for two hours. 0.12 parts by weight ultra high molecular weight polyethylene (UHMWPE) treated with oxygen and fluorine are added to the blend and dispersed by tumbling end-over-end in a fiber drum.

C. Colored and uncolored blends are blended to produce a shaded powder blend by tumbling end-over-end in a fiber drum.

D. A liquid blend is formed by mixing 657 g methyl methacrylate (a monomer) 138 g Bis-phenol-A-Dimethacrylate (a crosslinker), 200 g 2,4,4-trimethyl-1,4-diurethanedimethacrylate hexane (methacrylated end capped) (a second crosslinker) and 5 g Benzoyl Peroxide (initiator) in a large polyethylene carboy for 30 minutes.

E. A precursor blend was formed by mixing 570 grams of the powder blend, C above, with 430 grams of the liquid blend, D above, in a planatary mixer. The wet mixture is poured through a spout into polyethylene sleeves which are from 2 and 3/8 inch to 4 and 1/4 inch in diameter. the sleeves are hung vertically until the wet mixture solidifies to a viscosity (determined by a penetrometer reading) between 40 and 50 for tooth enamel material and between 55 and 65 for tooth body material. The sleeves are placed in a freezer for 16 hours and then the tooth material is sliced to 1/8 inch for tooth enamel and 1/4 inch for tooth body. The sliced material is aged for a minimum of 3 days in a freezer and is then molded and heated to 100°C to form a posterior tooth.

The tooth was evaluated for wear resistance in a computer programmed chewing device (Journal of Prosthetic Dentistry, Volume 54, Number 2, August 1985, pages 273 - 280). It demonstrated a volume loss from wear against a natural tooth of .021 $mm^3$, compared to 0.032 $mm^3$ loss for commercial tooth, BIOFORM (Dentsply) prepared in general according to U.S. Patent 4,698,373.

EXAMPLE 5

TOOTH HAVING SELF-LUBRICATING SILICON CONTAINING MATERIAL

The procedure of Example 4 is followed except that liquid blend is formed by mixing 795 g methyl methacrylate (a monomer) 175 g Bis-phenol-A-Dimethacrylate (a crosslinker) 25 g divinyldimethylsilane (a second crosslinker) and 5 g Benzoyl Peroxide (initiator).

The tooth was evaluated for wear resistance in a computer programmed chewing device (Journal of Prosthetic Dentistry, Volume 54, Number 2, August 1985, pages 273 - 280). It demonstrated a volume loss from wear against a material tooth of .023 $mm^3$. Compared to 0.032 $mm^3$ loss for commercial tooth, BIOFORM (Dentsply) prepared in general according to U.S. Patent 4,698,373.

It should be understood that while the present invention has been described in considerable detail with respect to certain specific embodiments thereof, it should not be considered limited to such embodiments but may be used in other ways without departure from the spirit of the invention and the scope of the appended claims.

**Claims**

**1.** Artificial tooth dental prosthesis comprising:

particles of a polymer of the general formula:

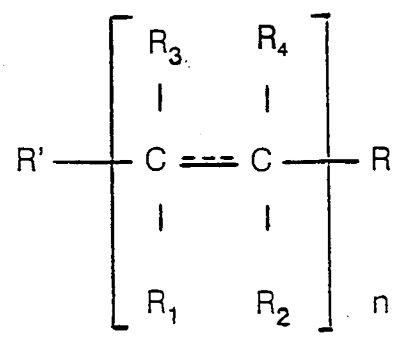

wherein n is an integer from 100 to 1,000,000, - - - is a single or a double bond, when - - - is a double bond R, R', $R_1$, $R_2$ independently are hydrogen, fluorine or a lower alkyl of from 1 to 6 carbons, and $R_3$ and $R_4$ are not present; when - - - is a single bond R, R', $R_1$, $R_2$, $R_3$ and $R_4$ independently are hydrogen, fluorine or a lower alkyl of from 1 to 6 carbons, within a polymer matrix.

**2.** The artificial tooth dental prosthesis of Claim 1 wherein said self-lubricating particles comprise polyethylene having a molecular weight of at least 1,000,000, said polyethylene having a coefficient of friction less than about 0.25.

**3.** The artificial tooth dental prosthesis of Claim 2 wherein said polyethylene has a molecular weight greater than 1,000,000 and is in the form of particles having a particle size less than 80 microns, and are chemically bonded to said polymeric matrix.

**4.** The artificial tooth dental prosthesis of Claim 1 wherein said matrix is an interpenetrating polymer network.

## Patentansprüche

**1.** Künstlicher Zahnersatz, umfassend:

Teilchen eines Polymers der allgemeinen Formel:

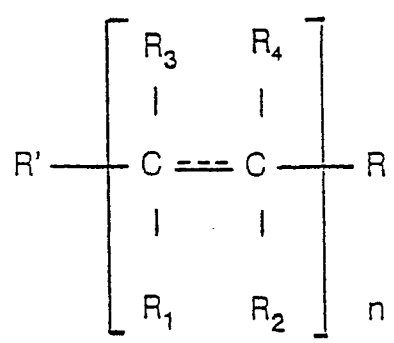

in der n eine ganze Zahl von 100 bis 1.000.000 ist, - - - eine Einfach- oder Doppelbindung ist, wenn - - - eine Doppelbindung ist, R, R', $R_1$ und $R_2$ unabhängig Wasserstoff, Fluor oder ein Niederalkyl mit 1 bis 6 Kohlenstoffen sind und $R_3$ und $R_4$ nicht anwesend sind; wenn - - - eine Einfachbindung ist, R, R', $R_1$, $R_2$ , $R_3$ und $R_4$ unabhängig Wasserstoff, Fluor oder ein Niederalkyl mit 1 bis 6 Kohlenstoffen sind, in einer Polymermatrix.

**2.** Künstlicher Zahnersatz nach Anspruch 1, in dem die selbstschmierenden Teilchen Polyethylen mit einem Molekulargewicht von mindestens 1.000.000 umfassen, wobei das Polyethylen einen Reibungskoeffizienten von weniger

als etwa 0,25 aufweist.

3. Künstlicher Zahnersatz nach Anspruch 2, in dem das Polyethylen ein Molekulargewicht von mehr als 1.000.000 aufweist und in Form von Teilchen vorliegt, die eine Teilchengröße von weniger als 80 µm aufweisen und chemisch an die Polymermatrix gebunden sind.

4. Künstlicher Zahnersatz nach Anspruch 1, in dem die Matrix ein interpenetrierendes Polymernetzwerk ist.

**Revendications**

1. Prothèse dentaire formée d'une dent artificielle, comprenant :

des particules d'un polymère répondant à la formule générale :

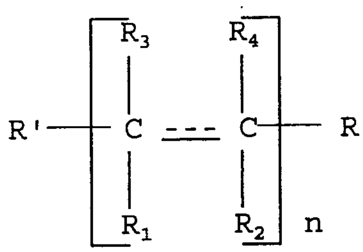

où n est un nombre entier de 100 à 1 000 000,
--- est une liaison simple ou une liaison double;
quand --- est une liaison double, R, R', $R_1$ et $R_2$ sont indépendamment l'hydrogène, le fluor ou un groupe alkyle inférieur ayant de 1 à 6 atomes de carbone, et $R_3$ et $R_4$ ne sont pas présents ; quand --- est une liaison simple, R, R', $R_1$, $R_2$, $R_3$ et $R_4$ sont indépendamment l'hydrogène, le fluor ou un groupe alkyle inférieur ayant de 1 à 6 atomes de carbone, à l'intérieur d'une matrice polymère.

2. Prothèse dentaire formée d'une dent artificielle selon la revendication 1, dans laquelle lesdites particules autolubrifiantes comprennent du polyéthylène ayant un poids moléculaire d'au moins 1 000 000, ledit polyéthylène ayant un coefficient de frottement inférieur à environ 0,25.

3. Prothèse dentaire formée d'une dent artificielle selon la revendication 2, dans laquelle ledit polyéthylène a un poids moléculaire supérieur à 1 000 000 et est sous la forme de particules ayant une dimension inférieure à 80 micromètres et qui sont liées chimiquement avec ladite matrice polymère.

4. Prothèse dentaire formée d'une dent artificielle selon la revendication 1, dans laquelle ladite matrice est un réseau polymère à interpénétration.

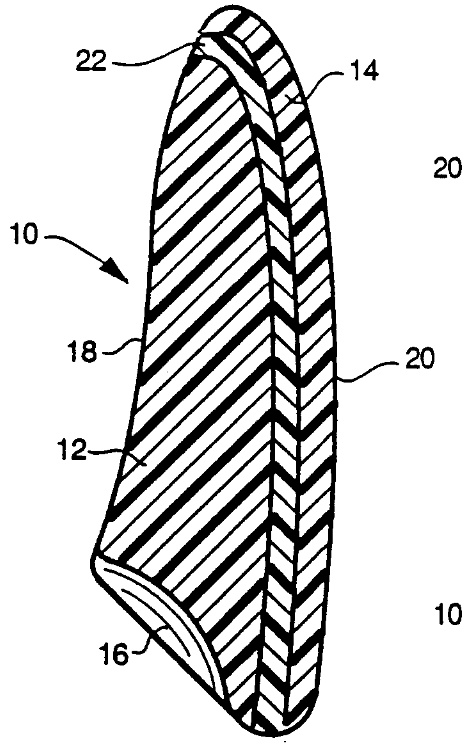


Fig. 1

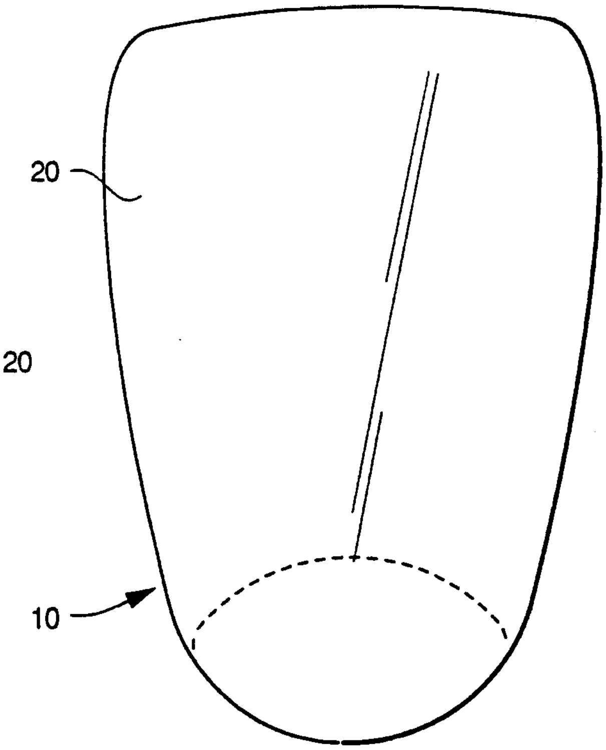


Fig. 2

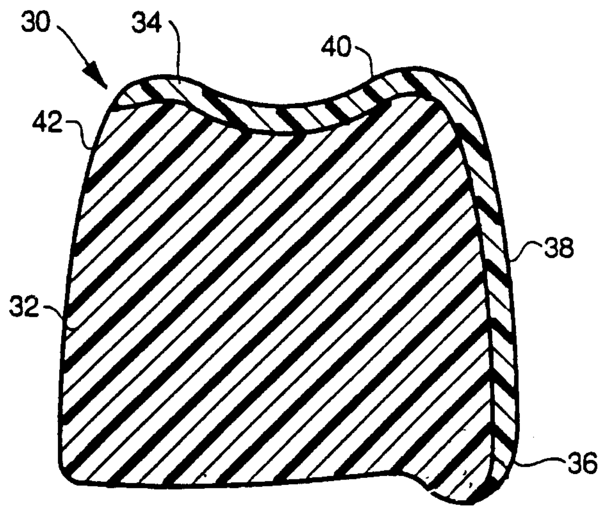


Fig. 3

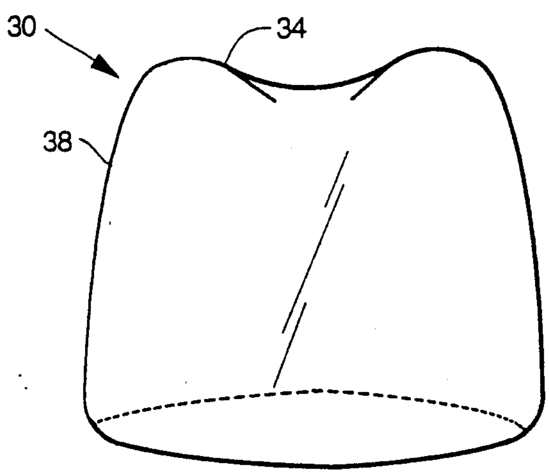


Fig. 4